Europäisches Patentamt

European Patent Office

Office européen des brevets

(11) Publication number: **0 486 020 A2**

# EUROPEAN PATENT APPLICATION

(21) Application number: **91119448.8**

(22) Date of filing: **14.11.91**

(51) Int. Cl.⁵: **C08K 3/00**, C08G 18/10, A61L 15/07

(30) Priority: **16.11.90 US 614433**

(43) Date of publication of application:
**20.05.92 Bulletin 92/21**

(84) Designated Contracting States:
**CH DE ES FR GB IT LI NL**

(71) Applicant: **MINNESOTA MINING AND MANUFACTURING COMPANY**
**3M Center, P.O. Box 33427**
**St. Paul, Minnesota 55133-3427(US)**

(72) Inventor: **Scholz, Matthe T., c/o Minnesota Mining and**
**Manufact. Co., 2501 Hudson Road, P.O. Box 33427**
**St. Paul, Minnesota 55133-3427(US)**

(74) Representative: **Vossius & Partner**
**Siebertstrasse 4 P.O. Box 86 07 67**
**W-8000 München 86(DE)**

(54) Luminescent water-curable materials.

(57) Disclosed is a composition containing a water-curable, isocyanate-functional resin and an inorganic phosphor.

EP 0 486 020 A2

The present invention relates to water-curable, isocyanate-functional resins. In particular, the present invention relates to water-curable, isocyanate-functional resins useful in orthopedic casting materials.

Water curable, isocyanate functional resins are known as coatings in formulating rigid objects, such as orthopedic casting materials, as disclosed, for example, in U.S. Patent No. 4,502,479 (Garwood et al.), U.S. Patent No. 4,376,438 (Straube), U.S. Patent No. 4,411,262 (VonBonin), and U.S. Patent No. 4,667,661 (Scholz et al.). In orthopedic casts, typically a knitted fiberglass or polyester fabric is used as the scrim onto which such resins are coated.

Since curing of such substrate-coated materials is initiated by water, they are routinely stored in moisture-proof pouches or containers. Since materials such as water-curable orthopedic casts are frequently stored for significant periods of time before use, storage stability is a significant concern.

Various colored resin-coated materials are also known. Klintworth (U.S. Patent No. 4,934,356) mixes a non-reactive pink or blue colorant of a limited class with a polyol to provide colored casting resins. Polta (U.S. Serial No. 376,565), now U.S. Patent No. 5,052,380, describes a variety of colors in casting resins wherein the colorant is covalently bound into the prepolymer resin.

The present invention relates to making water-curable, isocyanate-functional resins and articles made therewith luminescent. Accordingly, the present invention includes a composition comprising a water-curable, isocyanate-functional resin wherein the composition further comprises an inorganic phosphor.

Phosphors, also known as luminophors, are luminescent materials that, unlike conventional colorants, do not merely reflect light, but are actually light sources. They absorb radiant energy, usually in the ultraviolet range, i.e., below 4000 angstroms, and convert part of the absorbed primary energy into emitted luminescent radiation. Put another way, phosphors absorb radiant energy and convert the energy into longer visible wavelengths, emitting these longer wavelengths as visible light. Phosphors that cease to emit light when the source of radiant energy is removed are fluorescent phosphors, while phosphors that continue to emit light after the source of radiant energy is removed are phosphorescent phosphors, which glow in the dark. Preferably, the phosphor used in accordance with the presently claimed invention does not react with the resin, e.g., in any way that would undesirably increase the viscosity of the resin during storage, and does not leach out of the resin or migrate after mixing.

Phosphors are well known such as disclosed in Wanamaker et al., Progress in Organic Coatings, 3, - (1975) 305-322, the disclosure of which is incorporated herein by reference. Synthesis of useful phosphors is well known, such as disclosed in Leverenz, An Introduction To Luminescence Of Solids, John Wiley and Sons, Inc., New York (1950), the disclosure of which is incorporated herein by reference. Exemplary phosphors for use in accordance with the present invention include metal sulfides such as zinc sulfide, calcium sulfide, magnesium sulfide, strontium sulfide, and cadmium sulfide. Inorganic phosphors include materials in which activator cations, such as copper, silver, tin, cadmium, lead, manganese, and bismuth, are present. Such phosphors include copper activated zinc sulfide and cadmium activated zinc sulfide. In orthopedic cast formulations, it is, of course, important that the phosphor used be non-toxic. Commercially available phosphors that are preferred for use in accordance with the present invention include inorganic phosphorescent pigment 2205 blue (silver [less than 0.5%] activated zinc sulfide having a particle size of approximately 4 microns), fluorescent pigment 2210 green (copper [less than 0.5%] activated zinc sulfide), phosphorescent pigments EXCITE 2330 LBY (copper activated zinc sulfide, emission color green-blue, body color yellow-green, particle size 21-23 $\mu$m) and EXCITE 2330 MBW (copper activated zinc sulfide, emission color green-blue, body color white-green, particle size 18-22 $\mu$m), and EXCITE 2304 (cadmium activated zinc sulfide, orange daylight color, exhibits red phosphorescence), all of which are available from USR Optonix Inc., Hackettstown, New Jersey. Optionally, a well known colorant visible under ordinary light is also included in the composition of the present invention. Preferred commercially available colorants include RADIANT chartreuse pigment T1-CH 6720, RADIANT orange pigment T1-OG 6613, and RADIANT green pigment T1-Gr 6611, all of which are available from Radiant Color, Richmond, Virginia.

The preferred resins useful in accordance with the present invention are water curable, isocyanate-functional, polyurethane prepolymer resins which are formed by reaction of a polyol with an excess of a suitable polyisocyanate. An excess of the polyisocyanate is employed in formulating the prepolymer such that the resultant prepolymer contains unreacted isocyanate groups and is isocyanate functional, and is therefore capable of curing to a thermoset state upon contact with water. When used, for example, in an orthopedic casting material in accordance with the present invention, the phosphorescent orthopedic resin formulation is coated onto a suitable scrim. Although many scrims made from natural or synthetic yarns are known for such purposes, it is presently preferred to employ a knitted fiberglass or polyester fabric as the scrim material.

Preferred resins for use in the present invention include those disclosed in U.S. Patent No. 4,667,661 (Scholz et al.) and U.S. Patent No. 4,774,937 (Scholz et al.), the disclosures of which are incorporated herein by reference. The polyurethane prepolymer resins disclosed in these patents are formed by the reaction of a polyol with a polyisocyanate.

Conventional polyols which may be used in forming the polyurethane prepolymers of the present invention include polypropylene ether glycols (available from AC West Virginia Polyol Co., Lisle, Illinois as Niax™ PPG and from BASF Wyanadotte Corp., Michigan as XAS 10961.00 experimental polyol), polytetramethylene ether glycols (available from Quaker Chemical Company, Conshohocken, Pennsylvania as Polymeg™), polycaprolactone diols (available from Union Carbide as the Tone™ series of polyols) and polyester polyols (hydroxyl terminated polyesters obtained from the esterification of dicarboxylic acids and diols such as the Lexorez™ polyols available from Inolex Corp., Chemical Division, Philadelphia, Pennsylvania). As will be appreciated by those skilled in the art, the rigidity of the cured resin can be reduced by increasing the molecular weight of the polyols employed, or conversely, the rigidity can be increased by using lower molecular weight polyols.

It will be understood that, as used herein, the term "polyol" also includes virtually any functional compound having active hydrogen in accordance with the well-known Zerevitinov test, as described, for example, in Chemistry of Inorganic Compounds by Carl R. Noller, Chapter 6, pp. 121-122 (1957). Thus, for example, thiols and polyamines could also be used as "polyols" in the present invention, and the term "polyols" will be considered to include such other active hydrogen compounds.

In choosing an appropriate polyisocyanate, it is presently preferred to use an isocyanate which has a relatively low volatility, such as diphenylmethane diisocyanate (MDI), rather than a more volatile material such as toluene diisocyanate (TDI), which is also more toxic. Presently preferred isocyanates include aromatic isocyanates 4,4'-diphenylmethane diisocyanate, 2,4'-diphenylmethane diisocyanate, and mixtures of these isomers together with possible small quantities of 2,2'-diphenylmethane diisocyanate (typical of commercially available diphenylmethane diisocyanate). However, isocyanates such as aromatic polyisocyanates and their mixtures which are derived from phosgenation of the condensation product of aniline and formaldehyde may also be used.

One example of a presently preferred resin which may be used in the invention involves the reaction of a polyisocyanate known as Isonate™ 2143L (formerly known as Isonate 143L, a mixture containing about 73% MDI) which is available from the Dow Chemical Company, Midland, Michigan with a mixture of polypropylene oxide polyols which are available from AC West Virginia Polyol Co. and are known as Niax™ PPG 725 and PPG 425. To prolong the shelf life of the resin, it is also preferable to include from about 0.01% to about 1% by weight of benzoyl chloride or other suitable acidic stabilizer.

The preferred curable resins used in the present invention are polymerizable to a thermoset state. The resin used is preferably nontoxic in the sense that it does not give off significant amounts of toxic vapors, which, e.g., may be harmful to either the patient or the person applying the orthopedic casting material, and also in the sense that it does not cause skin irritation either by chemical irritation or by the generation of excessive heat during cure. Furthermore, in casting materials, the resin should sufficiently react with the curing agent (e.g., water) to ensure rapid hardening of the orthopedic casting material once it has been applied, but not so reactive that it does not allow for sufficient working time to apply and shape the orthopedic cast or splint. Initially, the orthopedic casting material must be pliable and conformable and should adhere to itself. Then in a short time following the completion of application, it should become rigid, or at least semi-rigid, and strong enough to support the loads and stresses to which the cast or splint is subjected by the activities of the wearer. Thus, the orthopedic casting material must undergo a change of state from a flexible condition to a relatively rigid condition in a matter of minutes.

If desired, the aforementioned preferred resins can be modified by the use of a polyol containing a stable dispersion of hydrophobic polymeric particles which serve to reduce foaming during cure. Polyurethane prepolymer resins are prepared by reacting a polyisocyanate with a polyol having polymeric particles dispersed therein which serve to reduce foaming during cure and provide other benefits. Preferably, such polymeric particles are made from hydrophobic vinyl monomers. However, any monomers may be employed which will form polymeric particles that act to significantly reduce foaming. Polymeric particles which have been found suitable for this purpose include polyacrylonitrile, a copolymer of acrylonitrile and styrene, and polyurea (formed, for example, from toluene diisocyanate and ethylenediamine). Polymeric particles made from epoxy based resins or combinations of any of the foregoing would also be suitable.

Several polyols are commercially available which already have such polymeric particles dispersed therein, and are thus suitable for practicing the invention. For example, Niax E-561 and E-562 polyols (available from AC West Virginia Polyol Co.) which contain polymeric particles made of a copolymer of acrylonitrile and styrene (in a 50/50 weight % ratio); Niax E-701 polyol (also available from AC West Virginia Polyol Co.) which contains polymeric particles made of polyacrylonitrile; and Multranol 9151 polyol (available from Mobay Chemical Corporation, Pittsburgh, Pennsylvania) which contains polymeric particles made of a polyurea; have been found to be useful in practicing the present invention. The presently most preferred polyols having polymeric particles dispersed therein are the aforementioned Niax E-562 and E-701 polyols, which are referred to by Union Carbide Corporation as "Niax Performance Polyether Polymer Polyols".

Preferably, the polyols contain polymeric particles having an average diameter of less than about 20 microns. Preferably, the average diameter of the polymeric particles is greater than about 0.01 microns to about 10 microns, and most preferably from about 0.3 microns to about 5 microns. However, there apparently is no minimum average diameter of particle needed to achieve the benefits of the invention, and it is believed that smaller particle sizes work best. Although polymeric particles sizes greater than the foregoing may be employed, it is believed that some of the benefits of the polymeric particles may be sacrificed if the particle sizes are too high.

Moreover, in the commercially available polymeric particles containing polyols mentioned herein, the polymeric particles comprise from about 20% to about 38% by weight of the polyol. It is believed that polyols containing 10% to 45% by weight function adequately. When incorporating such polymeric particle containing polyols into the curable resins of the present invention, the polymeric particles preferably comprise from about 0.5% to about 10% by weight of the resin, more preferably from about 1% to about 6% by weight of the resin, and most preferably from about 2% to about 4% by weight of the resin.

The preferred luminescent materials of the present invention are shelf stable, i.e., in the absence of water, over time. In addition, preferred phosphors disperse readily into the resin using conventional mixing equipment. Once the resin is coated into the scrim, no significant settling or migration of the phosphors is experienced, and excellent uniformity is achieved. Thus, the preferred luminescent materials of the present invention do not suffer from the problems of poorly mixed pigments. Further, the presence of metal sulfide phosphors causes no additional increase in viscosity of the resins over time, indicating no decrease in shelf life.

The compositions of the present invention have particular utility when used in orthopedic casting applications as well as in materials to protect the surface of an article or structure from abrasion or corrosion or as a material for reinforcing, sealing, or repairing the surface of an article or structure requiring such.

The phosphors and pigments used to color the resin mixtures and the casts formed from the resin mixtures are preferably added after all of the other ingredients have been added to the prereacted prepolymer resin and just before coating the resin mixture onto a fabric sheet and packaging the resin-coated fabric. Since the phosphors and pigments are relatively dense (e.g., about 4.1 g/cc) a thorough mixing is needed to obtain effective dispersal of the phosphors and if used, the pigments. A suitable mixer used to obtain good mixing of the phosphor is a 45 degree pitched 4 blade turbine impeller. If the Radiant pigment is added a higher shear mixer such as Premier Laboratory dispersator (2000 series) is preferred (available from Premier Mill Corp.).

Although the amount of phosphor and pigment employed in a particular resin will vary according to the type, shade, and intensity of the luminescence and color desired, typically the pigments comprise from about 0.01% to about 2% by weight of the total resin, more preferably from about 0.02% to about 1.0% by weight of the resin. The phosphors typically comprise from about 1% to about 12% by weight of the total resin, and preferably about 5%.

The combined phosphors and pigments of the present invention provide colored luminescent resins which are color stable, storage stable and exhibit persistent and stable brightness after being stored at the relatively high temperatures (about 38°C) at which the prepolymer resins are generally stored before being coated onto a suitable scrim for use as an orthopedic casting material. The resultant colored luminescent casting material is then stored in a moisture-free pouch or container, under which conditions the colored luminescent casting material again is extremely color and luminescence stable. The color and luminescence of the resin-coated materials of the present invention remains stable through the period of removing the casting material from the pouch, activating the material by dipping it in water, and allowing the material to cure.

As is evidenced by the appended examples, the phosphors and pigments of the present invention provide a colored luminescent resin which is storage stable in the sense that the uniformity of color is quite good and remains substantially unchanged after about 5 weeks of aging at 49°C. Further, the resin-coated materials are storage stable in the sense that the presence of the phosphors and pigments do not cause a significantly higher rate of viscosity increase in the resin over time as compared to noncolored resins. More particularly, storage stable resins are those, which when coated on a suitable scrim, packaged, and stored in a sealed container at 150°F for 7 days, preferably 14 days, more preferably 21 days, still provide a usable material.

In choosing the relative proportions of the resin ingredients for purposes of the present invention, the following criteria should be kept in mind: 1) proper ring strength and ring delamination strength of the resultant cast should be preserved by ensuring sufficient resin content, while at the same time preserving adequate porosity, when tested according to known methods, the dry strength tested as described below is preferably 30 to 80 lbs. per inch width and more preferably 40 to 70 lbs. per inch width, and most preferably 50 to 70 lbs. per inch width; 2) the viscosity of the resin should be kept within a range that enables the resin-coated casting material to be easily unwound and applied to the patient, generally within the range of 10,000 to 300,000 centipoise when measured on a Brookfield RVT Viscometer using spindle #6 at a temperature of 23°C, more preferably within the range of 10,000 to 100,000 centipoise, and most preferably within the range of 20,000 to 100,000 centipoise; 3) the set time (time until sufficiently cured to resist passive motion) of the material should be from about 2 to 18 minutes, more preferably from about 2.5 to about 10 minutes, and most preferably from about 3 to about 5 minutes.

In this test, the "dry strength" of certain cured cylindrical ring samples of the resin-coated materials of the present invention is determined. Each cylindrical ring comprises 6 layers of the resin-coated material having an inner diameter of 2 inches (5.08 centimeters). The width of the ring formed is the same as the width of the resin-coated material employed.

Each cylindrical ring is formed by taking a roll of the resin-coated material from its storage pouch and immersing the roll completely in deionized water having a temperature of about 80°F (27°C) for about 30 seconds. The roll of resin-coated material is then removed from the water and the material is wrapped around a 2 inch (5.08 centimeter) mandrel, covered with a thin layer of stockinet such as 3M Synthetic Stockinet MS02, to form 6 complete uniform layers using a controlled wrapping tension of about 45 grams per centimeter width of the material. Each cylinder is completely wound within 30 seconds after its removal from the water.

After 30 minutes from the initial immersion in water, the cured cylinder is removed from the mandrel, and allowed to cure for 24 hours in a controlled atmosphere of 75°F ± 3°F (34°C ± 2°C) and 55% ± 5% relative humidity. After this time, each cylinder is then placed in the Instron Model 1022 instrument fixture for testing. Compression loads are then applied to the cylindrical ring sample along its exterior and parallel to its axis. The cylindrical ring is placed lengthwise between the two bottom bars of the fixture (the bars being 1.9 centimeters wide, 1.3 centimeters in height, and 15.2 centimeters long), with the bars spaced about 4 centimeters apart. The inside edges of the bars are machined to form a curved surface having a 1/8 inch (0.31 centimeter) radius. A third bar (0.63 centimeters wide, 2.5 centimeters high, and 15.2 centimeters long) is then centered over the top of the cylinder, also parallel to its axis. The bottom or contacting edge of the third bar is machined to form a curved surface having a 1/8 inch (0.31 centimeter) radius. The third bar is brought down to bear against and crush the cylinder at a speed of about 5 cm/min. The maximum or peak force which is applied while crushing the cylinder is then recorded as the ring strength, which in this particular instance is the "dry strength" (expressed in terms of force per unit length of the cylinder). For each material, at least five samples are tested and the average peak force applied is then calculated. The reactivity of the curable resin, once it is exposed to the water, can be controlled by the use of a proper catalyst. The reactivity must not be so great that: (1) a hard film quickly forms on the resin surface preventing further penetration of the water into the bulk of the resin, or (2) the cast or splint becomes rigid before the application and shaping thereof has been completed. To produce suitable orthopedic casts and splints in accordance with the present invention, a set time of from about 2 to about 18 minutes following activation of the curable resin is preferred, with a more preferable set time being from about 2.5 to about 10 minutes, and a most preferable set time being from about 3 to about 5 minutes. Thus, the curable resins of the present invention also preferably contain a catalyst to control the set time and cure time of the resin.

Suitable catalysts for moisture curing polyurethane prepolymer resin systems are well known. Preferred catalysts include tertiary amine catalysts, such as 4-[2-[methyl-2-(4morpholinyl)ethoxy]ethyl] morpholine (MEMPE), disclosed in U.S. Patent No. 4,705,840 (Buckanin), the disclosure of which is incorporated herein by reference. The catalyst is used in amounts ranging from about 0.5% to about 5% by weight of the resin system.

If desired, tack reduction in casting materials can be achieved by lightly coating the surfaces of the resin-coated scrim with a mixture of a polydimethylsiloxane, having a viscosity of at least about 100 centistokes, and polyethylene oxide long chain aliphatic hydrocarbon waxes. More preferably, a small amount of a polyethylene oxide-polypropylene oxide block copolymer (such as Pluronic F-108 available from BASF Wyandotte) may be added to the resin during prepolymer preparation, after which, if desired, the polydimethylsiloxane may be applied onto the surface of the orthopedic article as before. The polydimethylsiloxane reduces resin tackiness prior to contact with water. The hydrophilic polyethylene oxide materials provide additional tack reduction upon contact with water.

Foaming of the resin-impregnated material (upon immersion in water) which would reduce the porosity and strength of the cured material and its overall strength can also be minimized by using a foam suppressor such as DB-100 silicone fluid (Dow Corning) (now believed to be available under a new name, namely, Dow Corning Antifoam 1400), silicone Antifoam A (Dow Corning, Midland, Michigan), or silicone surfactant L550 or L5303 (available from Union Carbide) to the resin. It is presently preferred to use the Dow Corning DB-100 silicone fluid (or Dow Corning Antifoam 1400) at a concentration of about 0.1% to about 1% by weight of the resin.

The preparation of the orthopedic casting materials of the present invention generally involves the simple coating of the curable resin onto the fabric scrim in a low humidity environment. Generally, the scrim should be resin loaded to the point where the resin represents from about 35% to about 80% by weight of the total weight of the resin-coated scrim. In the case of a fiberglass scrim, the resin preferably represents from about 35% to about 60% by weight of the total weight of the resin-coated scrim, and preferably from about 38% to about 45% by weight. Manual or mechanical manipulation of the resin (such as by a nip roller or wiper blade) into the scrim is usually not necessary. However, some manipulation of the resin into the fabric may sometimes be desirable. Care should be given not to stretch the fabric scrim during resin coating, however, so as to preserve the stretchability of the material for its later application around the desired body part.

The curable resins of the present invention may be coated onto a variety of well-known scrims for use as orthopedic casting materials or for other applications. Although many materials are well known for this purpose, fiberglass is presently preferred. In this regard, in one presently preferred embodiment of the present invention, the scrim comprises an extensible, heat-set, knitted fiberglass fabric as set forth in U.S. Patent No. 4,609,578 (Reed), the disclosure of which is incorporated herein by reference.

If desired, a series of projections may also be formed along such a knitted fiberglass scrim in order to enhance the lamination properties thereof. Such projections can be formed by abrading the scrim with, for example, a knurled roller, a knife edge, sharp or blunt teeth, or by other techniques. A projection is considered to be a filament bundle which serves to enhance lamination and typically has 8 or more filaments per bundle. The fabric scrims preferably have from about 75 to about 1,500 projections per gram of fabric scrim on the average, more preferably from about 100 to about 1,000 projections per gram of fabric scrim, and most preferably from about 300 to about 700 projections per gram of fabric scrim.

Orthopedic casting materials prepared in accordance with the present invention are applied to humans or other animals in the same fashion as other known orthopedic casting materials. First, the body member or part to be immobilized is preferably covered with a conventional cast padding and/or stockinet to protect the body part. Next, the curable resin is activated by dipping the orthopedic casting material in water. Excess water may then be squeezed out of the orthopedic casting material, and the material is wrapped or otherwise positioned around the body part so as to properly conform thereto. Preferably, the material is then molded and smoothed to form the best fit possible and to properly secure the body part in the desired position. Although often not necessary, if desired, the orthopedic casting material may be held in place during cure by wrapping an elastic bandage or other securing means around the curing orthopedic casting material. When curing is complete, the body part is properly immobilized within the orthopedic cast or splint which is formed.

The following non-limiting examples are provided in order to illustrate the present invention. All parts and percentages are by weight unless indicated otherwise.

Examples 1 and 2

In Examples 1 and 2, luminescent resins incorporating metalsulfide phosphors are prepared in accordance with the present invention. First, a 3200 g batch of a water curable, isocyanate functional, polyurethane prepolymer resin is prepared under dry conditions and constant agitation by adding the following chemical ingredients listed in Table I below in the proportions indicated therein.

TABLE I

| Ingredient | Weight % |
|---|---|
| Isonate 2143L isocyanate (Dow Chemical) | 56.22 |
| MEMPE catalyst (From U.S. Patent No. 4,705,840) | 1.32 |
| Pluronic F-108 polyethylene oxide-polypropylene oxide block copolymer (BASF Wyandotte Corp.) | 4.00 |
| DB-100 silicone fluid (Dow Corning) | 0.18 |
| Benzoyl chloride | 0.05 |
| Ionol® butylated hydroxytoluene (BHT) (Shell Chemical) | 0.48 |
| PPG-425 polyol (Union Carbide) | 12.75 |
| PPG-725 polyol (Union Carbide) | 25.00 |

The isocyanate/hydroxyl ratio is 3.15 and the theoretical isocyanate equivalent weight of the prepolymer resin is 375 g per isocyanate equivalent.

The exotherm created by the reactive ingredients raises the temperature within the insulated reaction vessel from about 45°C to about 60°C during the procedure. After all ingredients have been included in the reaction mixture, the resultant resin is allowed to cool to about 27 to 38°C.

In each of Examples 1 and 2, a 3200 g sample of luminescent resin is prepared. For each of the Examples, a quantity of prepolymer resin is produced as with the resin batch of Table I and mixed with the phosphor set forth in Table II below, using the resin and phosphor amounts set forth in Table II. This mixing is achieved by constant agitation of the prepolymer resin and phosphor in a mixing vessel for at least about 45 minutes, such that the phosphor colorant is uniformly mixed into the prepolymer resin by visual observation.

TABLE II

| Example | Phosphor | Resin Weight (lb) | Phosphor Weight (lb) |
|---|---|---|---|
| 1 | EXCITE 2304 Red (Cadmium sulfide) | 0.95 | 0.05 |
| 2 | EXCITE 2330 (Zinc sulfide) | 0.95 | 0.05 |

The phosphor containing resin is mixed until use by placing the sealed resin in a one gallon jar and placing it on a roller-type mixer.

Examples 3 and 4

In Examples 3 and 4, luminescent casting materials are made using the luminescent resins prepared in Examples 1 and 2, respectively. In each of Examples 3 and 4, the respective luminescent prepolymer resin is coated on an extensible, heat-set, knitted fiberglass fabric roll as disclosed in the EXAMPLE of U.S. Patent No. 4,609,578 (available from 3M Corporation under the name SCOTCHCAST 2 knitted fiberglass scrim), which is 3 inches (7.62 cm) wide and 675 feet (206 meters) long. Each phosphor-containing resin is applied at a coating weight of about 42.5% resin, and each 675 foot roll is then converted into 4 yard (3.66 meter) rolls which are wound around 0.75 inch (1.91 cm) diameter polyethylene cores and stored in moisture-proof pouches.

Even after about 8 months of aging in the pouch, upon removal, the color in each of the orthopedic casting materials of Examples 3 and 4 is still very uniform and substantially unchanged. Moreover, upon water activating the luminescent orthopedic casting materials of Examples 3 and 4 by dipping them in water, no significant leaching, migration, or bleeding of colorant from the resin is observed. The casting materials harden properly and function well as weight-bearing orthopedic casts. No sulfide odor is detected. Both of the casting tapes are found to exhibit a marked luminescence such that when activated by natural room lighting (fluorescent lights) the materials have a bright glow in a dark room.

Examples 5-7

In Examples 5-7 resins incorporating both phosphors and colorants. Example 5 employs a chartreuse colorant (RADIANT T1-CH 6720). Example 6 contains an orange colorant (RADIANT T1 OG 6613). Example 7 contains a green colorant (RADIANT T1 GR 6611).

In each of Examples 5-7, a water curable, isocyanate functional, polyurethane prepolymer resin is prepared under dry conditions by purging with nitrogen and constant agitation by adding the following chemical ingredients listed in Table III below, with each successive ingredient being added at about 5 minute intervals. The pigments are dried overnight in a 250°F oven and added last.

TABLE III

| (EXAMPLES 5-7) | | |
|---|---|---|
| Ingredient | Weight (%) | Weight (g) |
| Isonate 2143L | 55.41 | 1939.18 |
| Benzoyl Chloride | 0.05 | 1.75 |
| DB-100 | 0.18 | 6.30 |
| Ionol | 0.47 | 16.45 |
| MEMPE | 1.29 | 45.15 |
| PPG-425 | 15.95 | 558.33 |
| PPG-725 | 17.31 | 605.91 |
| Pluronic F-108 | 4.04 | 141.40 |
| Colorant | 0.30 | 10.50 |
| Phosphor 2330 Green (USR Corp.) | 5.00 | 175.00 |
| TOTAL | 100.00 | 3500.00 |

The resins of Examples 5-7 are then coated as described in Examples 3 and 4 onto an identical fabric roll tape and packaged in foil pouches. The tapes of Examples 5-7 are cured on a 2 inch (5 cm) diameter mandrell to form approximately 10 inch (35.4 cm) long cylinders. This is done by removing the roll, dipping tape in 23-25°C water, wrapping it about the mandrell and allowing it to cure. Each of the examples are pastel colored in normal light and exhibited a bright phosphorescent glow in a dark room. There is no sulfide odor and the casts exhibited normal set time, curing and unwinding handling properties.

Example 6 and Comparative Example 7

Replicates of resin coated tapes are prepared according to Example 2 wherein one set contained phosphor and the other set, prepared for comparative purposes, does not. The tape rolls are sealed in moisture proof pouches and placed in a 150°F (65.6°C) oven. After removing the rolls from the pouches, the force necessary to unwind the rolls is measured (using an Instron Model 1022) as a function of time, as shown in Table IV below. Each value represents an average of five (5) samples.

TABLE IV

| Time (in days) | Example 6 (in lb) contains 5% pigment 2330 | Comparative Example 7 (in lb) |
|---|---|---|
| 0 | 2.03 | 1.64 |
| 7 | 2.40 | 2.51 |
| 14 | 4.03 | 3.74 |
| 21 | 4.65 | 4.98 |
| 28 | 6.60 | 6.60 |
| 35 | 8.96 | 9.50 |

The rate of increase in the unwind force for the tape rolls is approximately the same. By statistical evaluation the linear regression slope is 0.195 for Example 6 and 0.216 for Comparative Example 7. This result indicates that the presence of the phosphor does not have a statistically significant adverse effect on the aging of the resin-coated tape.

Examples 8 and 9

Using 200 g samples of the resin of the type used in Example 1, 5% by weight of phosphor green (pigment 2330, USR Corporation) is mixed with 0.04 weight percent of MILLIKEN yellow x 15 (a pigment available from Milliken Research Corporation, Inman, South Carolina) (Example 8) or 0.02 weight percent of BASF yellow (a pigment available from BASF Corporation). The resin is then coated at a loading of 42.5% on fiberglass tape as described in Examples 3 and 4 hereinabove and the tape rolls stored in moisture

9

proof pouches. Upon water activating the luminescent casting materials of Examples 8 and 9 by dipping them in water, the tapes harden properly. The tapes are examined in a dark room, and each is observed to glow.

**Claims**

1. In an article comprising a substrate coated with a water-curable, isocyanate-functional resin composition, the improvement wherein the composition further comprises an inorganic phosphor.

2. The article of claim 1 wherein the phosphor is phosphorescent.

3. The article of claim 1 wherein the phosphor is a metal sulfide.

4. The article of claim 1 capable of attaining a strength of 30-80 lb per inch width upon curing.

5. The article of claim 1 wherein the substrate is a fiber scrim.

6. The article of claim 1 wherein the substrate is a flexible material.

7. The article of claim 1 being capable of attaining a dry strength of about 30-80 lb per inch width.

8. An orthopedic casting material comprising a scrim and a phosphorescent resin coating on said scrim.

9. An orthopedic casting material as recited in claim 8, wherein said scrim is made from fiberglass or polyester fabric.

10. An orthopedic casting material as recited in claim 8, wherein said phosphorescent resin coating comprises a water-curable, isocyanate-functional resin and an inorganic phosphor.

11. An orthopedic casting material as recited in claim 10, wherein the inorganic phosphor is a metal sulfide present in an amount from about 1% to about 12% by weight of the resin.

12. An orthopedic casting material as recited in claim 10, wherein said resin is non-toxic.

13. An orthopedic casting material as recited in claim 10, wherein said phosphorescent resin coating further comprises polymeric particles capable of reducing foaming during cure.

14. An orthopedic casting material as recited in claim 10, wherein said phosphorescent resin coating further comprises a polyethylene oxide-polypropylene oxide block copolymer.

15. An orthopedic casting material as recited in claim 10, wherein said phosphorescent resin coating further comprises a pigment.

16. In a method of making an article comprising coating a substrate with a composition comprising a water-curable, isocyanate-functional resin, the improvement wherein the composition further comprises an inorganic phosphor.

17. The method of claim 16 wherein the substrate is a flexible material.

18. The method of claim 16 wherein the phosphor is a metal sulfide.

19. The method of claim 16 wherein the composition further comprises a curing catalyst and a lubricant.

20. The method of claim 16 wherein the phosphor is phosphorescent.

21. The method of claim 16 wherein the article is capable of attaining a dry strength of about 30-80 lb per inch width.